# DEMANDE DE BREVET EUROPEEN

(11) **EP 0 827 721 A1**
(43) Date de publication de la demande: **11.03.1998**
(21) Numéro de dépôt: 97870128.2
(22) Date de dépôt: 01.09.1997
(51) Int. Cl.: A61C 8/00

(54) **Implant dentaire**

(30) Priorité: 04.09.1996 BE 9600741
(71) Demandeur: Fumal, Jean-Marie, 4530 Vaux-et-Borset (BE)
(72) Inventeur: Fumal, Jean-Marie, 4530 Vaux-et-Borset (BE)
(74) Mandataire: Van Malderen, Joelle

(57) **Abrégé**

Implant dentaire (1), caractérisé en ce qu'il comporte une gaine implantaire (3) rigide, un axe (5) rigide et porteur de la restauration prothétique et un matériau élastique (9) rupteur de pression, séparant ladite gaine (3) dudit axe (5) ainsi que des moyens (7) assurant le maintien dans lequel la partie intraimplantaire de l'axe implantaire est prolongé au-delà du niveau de l'hypomochlion.

## Description

### OBJET DE L'INVENTION

La présente invention concerne un implant dentaire, plus précisément un implant endo-osseux.

De tels implants constituent une structure de formes variables et de compositions variables mais nécessairement biologiquement compatibles, qui est insérée mécaniquement dans l'os maxillaire ou mandibulaire et qui comporte une partie émergente, supra-gingivale, destinée à recevoir la restauration prothétique.

Au niveau de chaque organe dentaire, la sertissure gingivale assure normalement une barrière entre le milieu extérieur (cavité buccale septique) et le milieu intérieur.

Cette barrière, si elle est rompue, permet une infiltration d'origine externe d'où poche parodontale, alvéolyse et, in fine, explusion de l'organe dentaire.

La liaison entre la racine dentaire et l'os alvéolaire est quant à elle constituée de fibres collagènes et élastiques, richement vascularisées et innervées. En plus de sa fonction de liaison, il sert de tampon hydraulique (exsudation - résorption) amortisseur des forces mécaniques de la racine vers l'os alvéolaire.

L'os alvéolaire est le volume osseux dans lequel se situent les alvéoles dentaires c'est-à-dire les réceptacles des racines dentaires.

Eminemment malléable, son intégrité dépend de l'équilibre des forces s'exerçant sur les dents.

Si l'équilibre est respecté (répartition homogène des forces masticatoires sur l'ensemble de la denture), l'os alvéolaire conserve son intégrité suite à l'eutrophisme par stimuli mécaniques adéquats.

Si l'équilibre est rompu (forces masticatoires s'exerçant préférentiellement sur une dent ou un groupe de dents), ceci amène une résorption de l'os alvéolaire : surcharge articulaire provoquant une déstabilisation de la ou des dents concernées.

En l'absence de la dent, l'os alvéolaire se résorbe inexorablement par défaut de sollicitation mécanique.

Pour un individu déterminé, lors de l'implantation endo-osseuse, le modèle physiologique doit être bien entendu respecté au maximum.

Pour ce qui concerne l'implant, les dimensions dentaires doivent être également respectées c'est-à-dire que le volume osseux disponible doit être suffisant tant verticalement que latéralement, pour accueillir un implant.

Cet implant lui-même doit présenter une dimension compatible avec le volume osseux.

L'implant se différencie cependant de l'organe dentaire par l'absence de sertissure gingivale, l'absence de ligament alvéolo-dentaire et le déplacement de l'axe de rotation vers l'apex radiculaire.

Il en résulte une absence de barrière entre le milieu extérieur et le milieu intérieur d'où une diminution de l'herméticité et surtout une diminution notable de la capacité d'amortissement des forces masticatoires.

Les corollaires en sont d'une part une infiltration de produits septiques le long du fût implantaire entraînant d'une part des lésions gingivales et une résorption osseuse d'origine infectieuse et d'autre part,une résorption osseuse périimplantaire par surcharge mécanique.

### RESUME DE L'ETAT DE LA TECHNIQUE

Dans le document WO 93/17634 A, on propose une base pour des prothèses dentaires comportant un corps extérieur en matériau métallique biocompatible, pourvue d'une cavité intérieure recevant un corps intérieur également en un matériau métallique biocompatible avec interposition d'une couche d'un matériau à propriété d'absorption de choc.

Le corps intérieur comporte un siège pour maintenir la prothèse.

Un élément annulaire de maintien est vissé au côté de la cavité dans le corps intérieur et est pourvu d'un côté intérieur déformable entrant en contact avec une découpe annulaire correspondante du corps intérieur.

De plus, un moyen d'accrochage dans le tissu osseux est prévu sous forme d'une découpe à la base dudit corps extérieur.

Il convient tout d'abord de noter que ce moyen d'ancrage n'est pas nécessaire si un effet d'amortissement suffisant est réalisé et que la néo-formation osseuse autour de la gaine est tout à fait suffisante pour maintenir l'implant en place.

De plus, ce moyen d'ancrage ne permet pas de profiter de toute la hauteur de la gaine implantaire.

Il convient à cet égard de noter que la hauteur endo-osseuse disponible varie de ***0,8*** à 1,8 cm.

La configuration retenue dans ce document implique que l'amortissement, tant axial que latéral, sera réduit par le fait même que la partie à effet d'amortissement n'occupe qu'une partie de la hauteur disponible, ce qui rend la configuration nettement différente du niveau de l'hypomochlion, c'est-à-dire l'axe de rotation de la racine naturelle.

De plus, ce document prévoit la fixation de l'implant par un pas de vis prévu dans le corps intérieur.

Etant donné qu'il est généralement admis qu'on ne dispose que d'un diamètre de 0,4 cm pour recevoir la gaine implantaire, ce moyen de fixation apparaît insuffisant pour répondre aux efforts mécaniques masticatoires ou tout du moins rendrait nécessaire de réduire l'espace réservé au matériau amortisseur.

Il convient encore de noter que la configuration même de l'élément annulaire de maintien tel qu'il est conçu, ne permet pas de mouvement de latéralité de ***l'axe implantaire.***

Il ne permet pas non plus de repositionnement correct de la superstructure et, étant donné l'importance des forces masticatoires, il pourrait présenter un phénomène de dévissage.

En résumé donc, la configuration retenue est encombrante et peu adaptée à l'usage envisagé.

On peut formuler des critiques similaires à l'égard du document US 5 453 07 A en ce sens que, également dans ce dispositif, on se heurte à des problèmes d'encombrement et d'absence d'effet d'amortissement surtout latéral.

A nouveau, une ouverture disposée à l'extrémité apicale dont l'utilité est contestable entraîne le non respect d'une configuration correspondant à l'hypomochlion.

Dans la forme d'exécution prévue, un élément de maintien sphérique constitue l'extrémité inférieur de l'axe implantaire rendant peu efficace la configuration à l'égard des sollicitations latérales.

Le document EP 0 312 698 A décrit un système composé de deux cylindres emboîtés sans prévoir d'interposition entre eux d'un matériau amortisseur.

Uniquement un effet d'élasticité intrinsèque d'un des éléments cylindriques est mentionné.

Le document US 5 197 881 décrit un dispositif de maintien d'un élément intérieur ou axe implantaire (post) dans un élément extérieur (gaine), basé sur des protubérances prévues sur l'élément intérieur qui viennent se loger dans des évidements correspondants prévus sur l'élément extérieur, par un système à clavette

Dans le document DE-A-3 611 139, on décrit une racine dentaire artificielle implantée dans une cavité osseuse recevant une racine artificielle. Elle comporte une interposition directe entre la cavité et la racine d'un matériau réducteur de pression constituée d'une matière biocompatible, sans recours à une gaîne, ce qui en pratique entraîne de nombreuses difficultés et constitue une solution peu fiable.

En effet, les matériaux biocompatibles inducteur de pression sont des polymères de synthèse dont les propriétés physiques s'altèrent avec le temps d'autant plus rapidement qu'ils sont en contact avec le milieu biologique très agressif.

Le remplacement, inéluctable, de ce matériau exigerait une intervention chirurgicale qui, sans aucun doute, entraînerait une diminution du volume osseux disponible.

### BUT VISE PAR LA PRESENTE INVENTION

La présente invention vise à fournir une solution aux problèmes mentionnés.

L'invention vise en particulier à porter remède à la diminution de capacité du système amortisseur et d'offrir un joint périphérique plus efficace dans le cas d'un implant dentaire.

### ELEMENTS CARACTERISTIQUES DE LA PRESENTE INVENTION

L'implant dentaire selon l'invention comporte une gaine implantaire rigide, un axe rigide et porteur de la restauration prothétique et un matériau élastique rupteur de pression séparant ladite gaine dudit axe ainsi qu'un moyen assurant le maintien de ces différents éléments tout en permettant un effet d'amortissement tant axial que latéral grâce au fait que la partie intraimplantaire de l'axe implantaire est prolongé au-delà du niveau de l'hypomochlion (c'est-à-dire l'axe de rotation de la racine naturelle qui est situé à l'union des deux tiers cervicaux et du tiers apical).

Avantageusement, la partie intra-implantaire de l'axe implantaire présente un léger renflement au niveau de l'hypomochlion.

Les deux axes diamétraux de ce renflement ne sont pas identiques :
- pour répondre au modèle physiologique où les efforts exercés sont plus importants dans le sens palato- ou linguo- vestibulaire.
- pour le maintien en position correcte de l'axe implantaire et du matériau amortisseur lors de leur remplacement éventuel.

Le moyen assurant le maintien de ces différents éléments est de préférence constitué par un bouchon.

La gaine implantaire rigide constitue la partie de l'implant qui est positionnée dans l'os par alésage mécanique de celui-ci. Avantageusement, on évitera qu'elle soit entièrement endo-osseuse, en ce sens que sa partie supérieure doit effleurer au niveau de la gencive pour réaliser ainsi un implant trans-muqueux.

L'axe implantaire, le matériau élastique sont solidaires, c'est-à-dire qu'ils forment un tout pouvant être inséré ou retiré de la gaine implantaire notamment pour permettre le remplacement du matériau élastique usé.

Cette solidarisation de deux éléments de l'implant (gaine implantaire et axe implantaire et matériau élastique solidarisés) se fait avantageusement à l'aide du bouchon (troisième élément de l'implant), bien que d'autres solutions techniques et physiologiques puissent être envisagées.

Un dispositif du type fermeture baïonnette peut convenir pour la fixation du bouchon dans la gaine, le maintien en place de ce bouchon étant provoqué par l'élasticité du matériau élastique rupteur de pression.

Une variante d'exécution peut être constituée notamment par l'utilisation d'un matériau à mémoire de forme du type NITINOL® pouvant agir pour la fermeture en assurant l'étanchéité et la solidarisation.

L'invention sera décrite plus en détail en référence aux dessins annexés donnés uniquement à titre d'illustration sans caractère limitatif d'une forme d'exécution de l'invention.

### BREVE DESCRIPTION DES DESSINS

La figure 1 représente une vue en coupe schématique de l'implant selon l'invention;

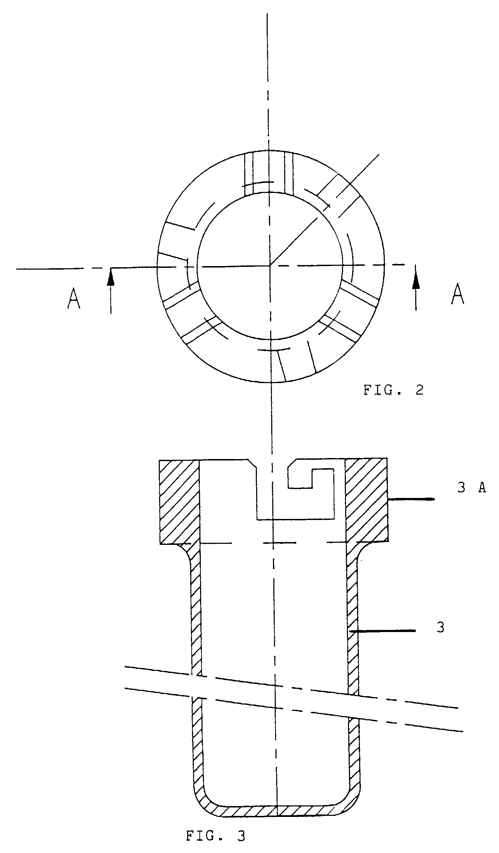

La figure 2 est une vue en plan de la gaine de la figure 1;
La figure 3 est une vue en coupe selon A-A de la figure 2;

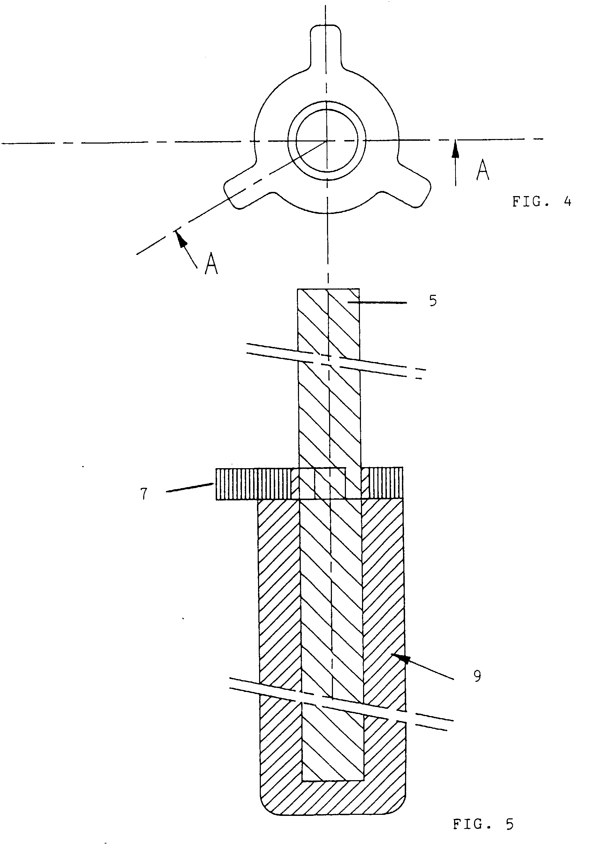

La figure 4 et une vue par le dessus de la figure 1, en l'absence de la gaine;
La figure 5 est une vue en coupe selon A-A de la figure 4;

Dans les différentes vues , des repères de référence identiques sont utilisés pour des éléments constitutifs identiques ou similaires.

L'invention sera décrite et illustrée en référence à une forme d'exécution préférée de l'invention, sans caractère limitatif.

### DESCRIPTION D'UNE FORME D'EXECUTION PREFEREE DE L'INVENTION

L'implant dentaire est représenté par le repère général 1. Il comporte une gaine 3 et un axe implantaire 5, représentés schématiquement sous forme cylindrique.

Les formes les plus adéquates à réaliser sont déterminées cependant par la finalité du projet, à savoir la répartition optimale des forces masticatoires au niveau du substrat osseux.

En ce qui concerne la gaine implantaire 3, les contraintes de l'alésage mécanique de l'os ne permettent pas beaucoup de variations dans sa forme externe. Les angles vifs sont en tout cas à éviter. La forme interne peut présenter certaines variations de diamètre.

Avantageusement, la partie intra implantaire de l'axe implantaire 5 présente un léger renflement 5' au niveau de l'hypomochlion.

Pour ce qui concerne la partie supra-implantaire de l'axe, il convient que l'axe implantaire 5 soit maintenu dans une position déterminée par rapport à la gaine implantaire 3 par le système de fermeture.

Sa position peut donc être déterminée lors de la mise en place de la gaine.

Pour les nécessités de l'occlusodontie, il est donc possible de produire des axes implantaires présentants une angulation à la jonction partie implantaire - partie supra-implantaire de l'axe.

Le bouchon 7 est le moyen de solidarisation mécanique le plus adapté entre la gaine implantaire 3 et le reste de l'implant.

Dans la phase de cicatrisation osseuse (laps de temps entre l'implantation de la gaine implantaire et la mise en charge prothétique de l'implant), il y aura une configuration cylindrique sans "jour" de sortie de l'axe implantaire.

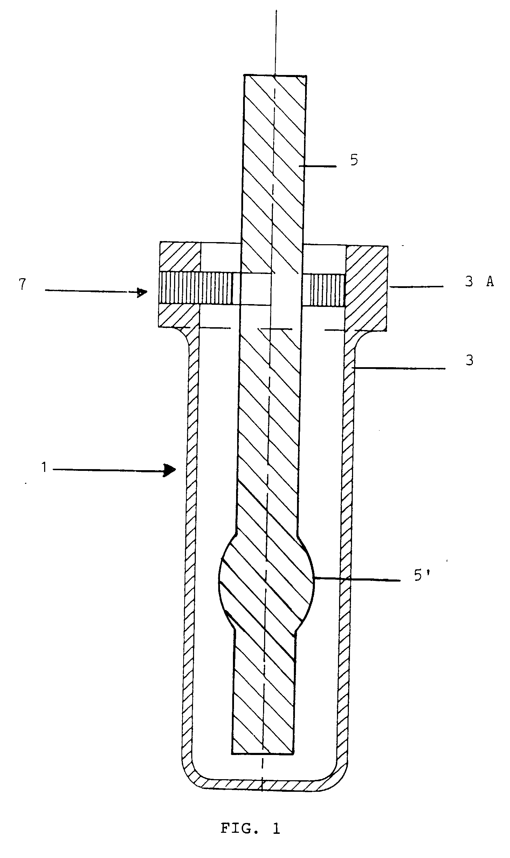

A sa partie apicale sera fixé le matériau dit rupteur de pression, c'est-à-dire un matériau élastique 9 assurant la coaptation gaine - bouchon (non représenté dans la figure 1).

Lors de la mise en charge de l'implant, le bouchon 7 comportera un jour de sortie en son centre permettant le passage de l'axe implantaire 5.

Le diamètre de ce jour sera supérieur au diamètre de l'axe implantaire afin de permettre les mouvements transversaux de celui-ci.

Pour la raison invoquée plus haut (remplacement de l'ensemble axe - bouchon - matériau réducteur de pression ou d'amortissement), la suprastructure prothétique doit être amovile, et ceci qu'il s'agisse d'une prothèse unitaire, d'un bridge ou d'une prothèse type amovo-inamobile.

Le système de fixation infrastructure - suprastructure vissé apparaît adéquat dans le cas présent.

Les matériaux choisis pour la gaine implantaire, l'axe et le bouchon sont de préférence le titane ou un alliage à base de titane.

Le titane et l'alliage Ti - Al sont déjà largement utilisés notamment en clinique dentaire et la tendance générale est de les préférer de plus en plus aux autres métaux.

En effet, le titane apparaît aujourd'hui comme le métal le plus résistant à la corrosion. C'est un métal hautement réactif et, c'est cette grande réactivité qui rend le métal si résistant à l'attaque par les environnements aqueux, car il est protégé par une couche de passivation d'oxyde de titane qui se forme spontanément.

Cette excellente résistance à la corrosion et le taux très bas de diffusion des ions associés à l'apparente absence d'effets biologiques du métal assure une bonne biocompatibilité.

Pour ce qui concerne le matériau rupteur de pression, les biomatériaux à base de polymères de synthèse - les polymères mous ou élastiques (élastomères) sont particulièrement indiqués. Les élastomères en effet, par la nature de leurs molécules à longues chaînes sont capables de résister à d'importantes déformations et de retourner à leur dimension originelle.

Conviennent tout particulièrement, la polydiméthylsiloxone (silicone) et le polyuréthanne (PU).

L'étude théorique de comportement permet de déterminer le choix de l'élastomère à utiliser compte tenu des propriétés physico-chimiques de celui-ci et des contraintes mécaniques et biologiques.

Dans la forme d'exécution donnée à titre d'exemple dans les dessins, la gaine 3 dans sa partie supérieure comporte un collet 3A dont l'épaisseur est supérieure à l'épaisseur de paroi de la partie inférieure de cette même gaine. Ce collet 3A est conformé avec trois logements par exemple, de manière à recevoir trois ergots fixés sur le bouchon et enfonçant le bouchon contre la résistance élastique de l'élastomère contenu dans la gaine et avec rotation.

L'implantation de la gaine dans l'os alvéolaire et sa fixation, s'effectuent de manière classique.

La technique proposée du point de vue physiologique se caractérise par deux facteurs essentiels, à savoir :
- la biocompatibilité, les matériaux utilisés pour la réalisation étant des matériaux ayant fait leur preuve et une adaptation éventuelle suite à l'avancée technologique restant toujours possible.
- la biofonctionabilité, c'est-à-dire la capacité qu'a un implant de faire face de façon effective et durable aux contraintes mécaniques, physiologiques et biologiques dans sa fonction de remplacement d'une structure naturelle.

En ce qui concerne l'organe dentaire, le but recherché est de créer un système ligamentaire, condition sine qua non à une répartition adéquate des forces masticatoires. Cette répartition amènera une diminution du risque de résorption osseuse par surcharge mécanique mais aussi, amènera une diminution de mobilité de la gaine implantaire et par conséquent, un rsique moindre de déchaussement gingival.

D'autre part, cet effet "ligament alvéolo-dentaire" permettra d'intégrer sans risque des éléments implantaires et naturels dans une même restauration prothétique.

Du point de vue pratique, en dehors de la mise en place de la gaine implantaire (infrastructure) qui demande un "routage" chirurgical, la technique utilisée est simple et accessible pour la mise en place et l'entretien de la méso-structure (bouchon - axe implantaire - matériau élastique) et de la superstructure (restauration prothétique).

Le système de solidarisation à baïonette est de plus un système simple.

En effet, pour la mise en place ou l'enlèvement de l'ensemble bouchon - axe implantaire - matériau élastique, il suffit d'une rotation d'un tiers de circonférence, d'où une facilité pour la mise en place de l'entretien.

Ce système permet également de repérer d'une façon tout à fait précise, la position relative de la gaine implantaire par rapport à l'ensemble bouchon - axe - matériau élastique. Il en résulte une facilité de repérage et de calcul d'une éventuelle angulation de l'axe implantaire à sa sortie du bouchon pour les nécessités de l'occlusodontie.

Dès lors que la situation de la position supra gingivale de l'axe prothétique est correcte, la confection de la superstructure prothétique se fait de façon classique. Sa fixation sur l'axe se fera par vissage palato ou linguo - vestibulaire afin d'en permettre la dépose.

## Revendications

1. Implant dentaire (1), caractérisé en ce qu'il comporte une gaine implantaire (3) rigide, un axe (5) rigide et porteur de la restauration prothétique et un matériau élastique (9) rupteur de pression, séparant ladite gaine (3) dudit axe (5) ainsi qu'un moyen (7) assurant le maintien dans lequel la partie intraimplantaire de l'axe implantaire est prolongé au-delà du niveau de l'hypomochlion.

2. Implant selon la revendication 1, caractérisé en ce que la partie intra-implantaire de l'axe implantaire présente un léger renflement (5') au niveau de l'hypomochlion.

3. Implant selon la revendication 1 ou 2, caractérisé en ce que le moyen assurant le maintien est constitué par un bouchon (7)

4. Implant selon la revendication 1, 2 ou 3, caractérisé en ce que la gaine (3) est conçue de manière à éviter qu'elle soit entièrement endo-osseuse, en ce sens que sa partie supérieure doit effleurer au niveau de la gencive pour réaliser ainsi un implant trans-muqueux.

5. Implant selon l'une quelconque des revendications 1 à 4, caractérisé en ce que l'axe implantaire (5) et le matériau élastique (9) sont solidaires, c'est-à-dire qu'ils forment un tout pouvant être inséré ou retiré de la gaine implantaire (3) notamment pour la période d'ostéointégration et le remplacement du matériau élastique usé.

6. Implant selon la revendication 5, caractérisé en ce que ladite solidarisation se fait à l'aide du bouchon (7)

7. Implant selon la revendication 6, caractérisé en ce que le bouchon (7) est fixé dans la gaine (3) par un dispositif du type fermeture baïonnette, le maintien en place de ce bouchon (7) étant provoqué par l'élasticité du matériau élastique rupteur de pression (9).

8. Implant selon la revendication 6, caractérisé en ce que le bouchon (7) est fixé dans la gaine (3) par l'utilisation d'un matériau à mémoire de forme du type NITINOL® qui assure l'étanchéité et la solidarisation.
